# EUROPÄISCHE PATENTANMELDUNG

(11) **EP 4 299 581 A1**
(43) Veröffentlichungstag der Anmeldung: **03.01.2024**
(21) Anmeldenummer: 22182374.3
(22) Anmeldetag: 30.06.2022
(51) Int. Cl.: C07K 5/062

(54) **VERFAHREN ZUR HERSTELLUNG VON DL-METHIONIN UND ZUR DIASTEREOMERENREINEN HERSTELLUNG VON DLLD-METHIONYL-METHIONIN**

(71) Anmelder: Evonik Operations GmbH, 45128 Essen (DE)
(72) Erfinder: KOBLER, Christophn, 63755 Alzenau (DE); HÄUSSNER, Thomas, 63808 Haibach (DE); BRAUNE, Sascha, 69221 Dossenheim (DE)
(74) Vertreter: Evonik Patent Association

(57) **Zusammenfassung**

Die Erfindung betrfft ein Verfahren zur Gewinnung von diastereomerenreinem DLLD-Methionylmethionin, dadurch gekennzeichnet, dass man eine das Natrium- und/oder Kaliumsalz von DLLD-Methionyl-methionin und das Natrium- und/oder Kaliumsalz von DDLL-Methionylmethionin enthaltende wässrige Lösung oder Suspension mit entsprechenden Mengen Kohlensäure und/oder CO2 versetzt (carbonisiert), so dass ein pH-Wert von 6 bis 9 gemessen mit einer Glaselektrode bei der sich einstellenden Temperatur erreicht wird, wobei ein überwiegend DLLD-Methionylmethionin enthaltender Niederschlag mit einem DLLD-Methionylmethionin-Anteil von mindestens 70 % gemessen am Gesamtgehalt von DLLD-Methionylmethionin und DDLL-Methionylmethionin ausfällt, der von der Mutterlauge, die DDLL-Methionylmethionin in angereicherter Form enthält, abgetrennt wird.

Dieses Verfahren ist Teil eines Gesamtverfahrens zur Herstellung von DLLD-Methionyl-methionin, welches aus dem primär erzeugten DLLD-Methionin-diketopiperazin (I) durch Hydrolyse erhalten wird sowie Teil eines Gesamtverfahrens zur gleichzeitigen Herstellung von DLLD-Methionylmethionin und DL-Methionin.

## Beschreibung

### Gebiet der Erfindung

Die vorliegende Erfindung betrifft ein Verfahren zur diastereomerenreinen Gewinnung von DLLD-Methionyl-methionin aus DLLD/DDLL-Methionyl-methionin-Gemischen und in diesem Zusammenhang auch ein Verfahren zur Herstellung von DLLD- Methionylmethionin im Verbund mit DL-Methionin enthaltend den Schritt der Cyclisierung und Epimerisierung **von** DDLL-Methionyl-methionin zum DLLD- 2,6-Bis(methionyl)-1,4-diketopiperazin (I, meso-Methionin-DKP, meso-*met-*DKP) und anschließender Hydrolyse.

Unter "diastereomerenrein" oder auch "im wesentlichen diastereomerenrein" wird in diesem Zusammenhang ein Produkt verstanden, das zumindest 90 Mol% des gewünschten DLLD-Diastereomeren enthält, vorzugsweise mindestens 95 bis 97 Mol% am Gesamtgehalt an enthaltenem Methionyl-methionin (Met-Met).

### Hintergrund der Erfindung

WO2010043558 A1 offenbart ein Verfahren zur Herstellung von Methionylmethionin (Met-Met), dem homologen Dipeptid aus DL-Methionin, welches vor allem als hochwertige Methioninquelle bereits bei der Fütterung von in Aquakulturen gehaltenen Fischen und Krustentieren zunehmend Anwendung findet. Aufgrund seiner zwei Stereozentren (zwei asymmetrische C-Atome) liegt das Produkt in Form von zwei Diastereomeren vor, dem DLLD-Met-Met und dem DDLL-Met-Met, also in Form von zwei konfigurationsisomeren Enantiomerenpaaren, die beide von den Tieren besonders gut verwertet werden können.

In der WO2010043558 A1 werden unterschiedliche Synthesemöglichkeiten zur Herstellung von Met- Met beschrieben. Allen Syntheseoptionen ist allerdings gemein, dass die Synthese von Met-Met über das homologe, cyclische Dipeptid des Methionins, das Methionin-diketopiperazin (I, Met-DKP oder DKP) erfolgt.

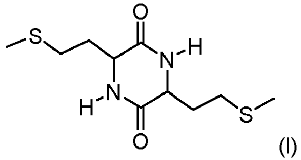

Die höchste Ausbeute an DKP von 78% bzw. 69% wird gemäß WO2010043558 A1 mit den zurzeit großtechnisch nicht zugänglichen Ausgangsmaterialen N-Carbamoylmethioninamid bzw. N-Carbamoylmethionin erzielt. Bei der Verwendung anderer Ausgangsstoffe, die großtechnisch verfügbar sind, wie z.B. Methionin-hydantoin (vgl.Schema 1) wird im besten Fall eine Ausbeute von immerhin 64% (Beispiel 7) beschrieben.

Allerdings führt die ringöffnende Hydrolyse von DKP mit Alkailauge, z.B. NaOH, zunächst zum Alkalisalz, z.B. Natriumsalz des Methionylmethionins, welches mittels Mineralsäure, z.B. H₂SO₄, zum freien Dipeptid Met-Met neutralisiert wird. Dieser Schritt führt zu äquimolaren Mengen Abfallsalz, z.B. Na₂SO₄, welches entsorgt werden muss. Dies führt zu einem unerwünschten Mehraufwand und ist belastend für Ressourcen und Umwelt.

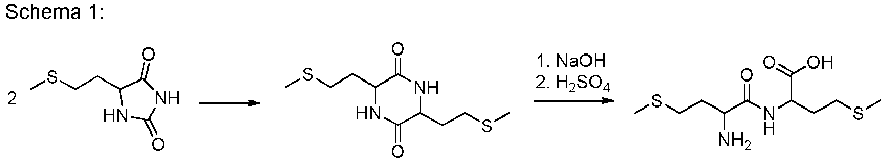

Die beiden bei der Herstellung zwangsläufig anfallenden Diastereomeren, dem DLLD-Met-Met und dem DDLL-Met-Met, können zwar beide von den Tieren gut verwertet werden, weisen aber deutlich unterschiedliche physikalische Eigenschaften auf, was in der praktischen Handhabung zu gewissen Problemen führen kann:
Es hat sich herausgestellt, dass die beiden Diastereomeren unterschiedliche Kristallstrukturen bilden. Während DLLD-Met-Met ein kugelförmiges Agglomerat in der Endproduktfällung bildet, kristallisiert DDLL-Met-Met in Nadeln aus. Damit sind unterschiedliche Rieselfähigkeit und Verklumpungsneigung sowie unterschiedliche Löslichkeit verbunden.

Während DLLD-Met-Met bei Raumtemperatur eine Wasserlöslichkeit von nur 0,4 g/l aufweist, liegt die Wasserlöslichkeit von DDLL-Met-Met mit 21,0 g/l viel höher. Eine hohe Wasserlöslichkeit ist aber für die Anwendung in Aquakulturen eher ungünstig, da es dadurch via Leaching aus dem Futterpellet zu Wertstoffverlusten kommen kann. Das Produkt aus dem Herstellungsprozess gemäß WO2010043558 A1 weist typischerweise ein Verhältnis von DLLD- / DDLL-Met-Met von 60 / 40 auf. Ein Produkt mit deutlich höherem Anteil an DLLD-Met-Met im Idealfall ein möglichst diastereomerenreines DLLD-Met-Met wäre daher wünschenswert.

Im Übrigen zeigt das bisher bekannte Produkt relativ niedrige Schüttgewichte von max. 500 kg/m³ , nur mäßige bis relative schlechte Rieselfähigkeiten von 3 (befriedigend) bis 5 (mangelhaft) und relativ niedrige Mindestzündenergie von MIE < 3 mJ, ist also vergleichsweise leicht entzündlich, so dass auch diese Eigenschaften verbesserungswürdig erschienen sind.

### Aufgabe

Es war daher Aufgabe der zugrundeliegenden Erfindung, ein Verfahren zur Herstellung einer weitgehend diastereomerenreinen DLLD-Met-Met- Qualität bereitzustellen, die also einen deutlich höheren Anteil von DLLD-Met-Met an der Gesamtmenge der Diastereomeren DLLD-Met-Met und DDLL-Met-Met im Vergleich zum Produkt gemäß des Stands der Technik aufweist. Eine weitere Aufgabe war, ein Verfahren bereitzustellen, das möglichst ohne den Zwangsanfall von Abfallsalz auskommt.

### Beschreibung der Erfindung

Die genannte Aufgabe wird mit der vorliegenden Erfindung gelöst, indem praktisch nur noch das weniger lösliche Enantiomerenpaar, das DLLD-Met-Met, isoliert wird und das unerwünschte DDLL-Met-Met wieder im Prozess verwertet wird, insbesondere in das unmittelbare Vorprodukt DKP zurückverwandelt wird und dabei simultan epimerisiert wird. Alternativ wird die Verwendung des unerwünschten DDLL-MetMets als Ausgangsstoff im Methioninprozess beschrieben.

Die der Erfindung zugrunde liegende Erkenntnis liegt darin, dass die Erfinder herausgefunden haben, dass beim Ansäuern bzw. Neutralisieren einer das Natrium- und/oder Kaliumsalz von DLLD-Methionyl-methionin und das Natrium- und/oder Kaliumsalz von DDLL-Methionyl-methionin enthaltenden wässrigen Lösung oder Suspension mit entsprechenden Mengen Kohlensäure und/oder CO₂ fast ausschließlich diastereomerenreines DLLD-Methionylmethionin als Niederschlag ausfällt, der sehr leicht abgetrennt werden kann, z.B. per Filtration oder Zentrifugation, während das besserlösliche DDLL-Methionyl-methionin in Lösung bleibt.

Einen grundlegenden Anteil an der Lösung der oben genannten Aufgaben liefert erfindungsgemäß ein

Verfahren zur Gewinnung von diastereomerenreinem DLLD-Methionylmethionin, dadurch gekennzeichnet, dass man eine das Natrium- und/oder Kaliumsalz von DLLD-Methionyl-methionin und das Natrium- und/oder Kaliumsalz von DDLL-Methionyl-methionin enthaltende wässrige Lösung oder Suspension mit entsprechenden Mengen Kohlensäure und/oder CO₂ versetzt (carbonisiert), so dass ein pH-Wert von 6 bis 9 gemessen mit einer Glaselektrode bei der sich einstellenden Temperatur erreicht wird, wobei ein überwiegend DLLD-Methionylmethionin enthaltender Niederschlag mit einem DLLD-Methionylmethionin-Anteil von mindestens 70% gemessen am Gesamtgehalt von DLLD-Methionylmethionin und DDLL-Methionylmethionin ausfällt, der von der Mutterlauge, die DDLL-Methionylmethionin in angereicherter Form enthält, abgetrennt wird.

Bevorzugt ist dabei ein Verfahren, bei dem so bzw.so lange carbonisiert, bis ein DLLD-Methionylmethionin-Anteil von mindestens 80 mol %, vorzugsweise 90 % im Niederschlag erhalten wird.

Besonders gute Diastereomerenreinheiten an DLLD-Methionylmethionin erhält man, wenn man bis zum Erreichen eines pH-Wertes von 6,4 bis 8,9, vorzugsweise von 7,0 bis 8,5, besonders bevorzugt **von 7,6** bis 8,5, insbesondere von 7,6 bis 8,2 carbonisiert. z.B von 96,9 bzw.97,4 mol% (vgl. Beispiele 1 und 2).

Dieser Effekt ist dem pH-Wert in Kombination mit der speziellen Matrixzusammensetzung der erzeugten Carbonisierungsmischung zuzuschreiben und als überraschend zu bezeichnen.

Vorzugsweise wird dabei die Carbonisierung bei einer Temperatur von 20 bis 50 °C, besonders bevorzugt bei 25 bis 40°C durchgeführt, was ebenfalls die hohe Diastereomerenreinheiten sowie auch gute Filtrierbarkeiten des abzutrennenden Niederschlags befördert.

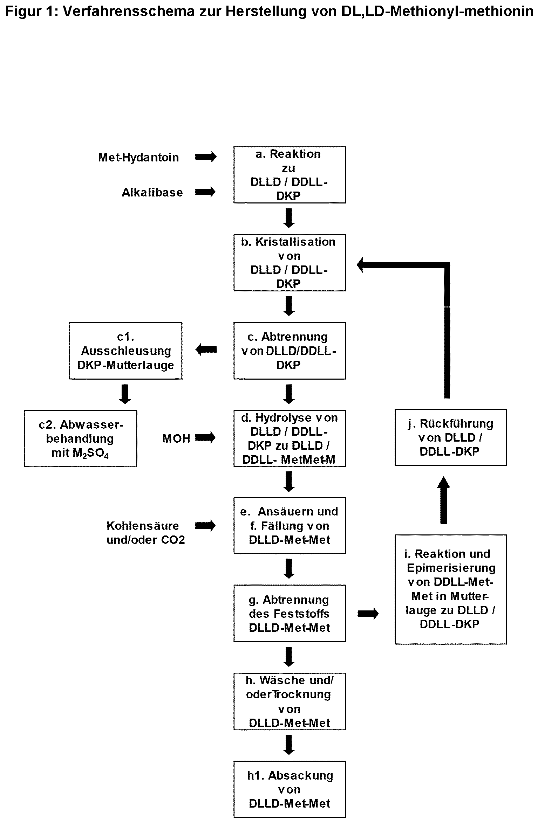

Ein weiterer Gegenstand der vorliegenden Erfindung und mit ihr in direktem Zusammenhang stehend ist das folgende Gesamtverfahren (vgl. Figur 1) zur Gewinnung von diastereomerenreinem DLLD-Methionylmethionin, dadurch gekennzeichnet, dass man
a. Methionin-Hydantoin zusammen mit Alkalibase umsetzt zu einem Diastereomerengemisch aus DLLD-Methionindiketopiperazin und DDLL-Methionindiketopiperazin (DLLD-DKP und DDLL-DKP)
b. das DLLD-DKP und DDLL-DKP aus a. durch Einengen oder Abkühlen der Reaktionslösung aus a. zur Kristallisation bringt
c. das auskristallisierte DLLD-DKP und DDLL-DKP aus b. von der DKP-Mutterlauge abtrennt
d. das abgetrennte DLLD-DKP und DDLL-DKP aus c. in Wasser einmischt und das Gemisch durch Zugabe entsprechender Mengen MOH (M= Alkali, z.B. Na und/oder K) auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DLLD-Methionylmethionin- und DDLL-Methionylmethionin-Alkalisalz hydrolysiert,
e. den pH-Wert der Lösung oder Suspension aus d., gemessen mit einer pH-Elektrode bei 20°C, durch Zugabe entsprechender Mengen Kohlensäure bzw. CO₂/H₂O auf pH 6 bis 9, vorzugsweise 6,4 bis 8,9 besonders bevorzugt 7,0 bis 8,5, ganz besonders bevorzugt 7,6 bis 8,5, insbesondere von 7,6 bis 8,2 absenkt gemäß der oben erläuterten zugrunde liegenden Erfindung (Ansäuern bzw. Carbonisieren),
f. durch Verdampfen von Wasser und/oder Abkühlen eine Suspension erzeugt (Fällung), die aus einem Feststoffanteil, der vorwiegend DLLD-Methionylmethionin enthält, und der Mutterlauge, die vorwiegend DDLL-Methionylmethionin enthält, besteht, man
g. den Feststoffanteil aus f. von der Mutterlauge abtrennt, und man
h. den abgetrennten Feststoffanteil aus g. wahlweise wäscht und/oder trocknet, und dabei diastereomerenreines DLLD-Methionylmethionin erhält und man
i. die verbleibende DDLL-Methionylmethionin enthaltende Mutterlauge aus g. gemäß der. zunächst durch Zufuhr von Kohlensäure und/oder CO2auf einen pH- Wert von 6,4 -7,5 bringt und dann bei einer Temperatur von 150 bis 220 °C mittels Cyclisierung umsetzt, so dass eine wässrige Lösung oder Suspension enthaltend ein Diastereomerengemisch aus DLLD-Methionindiketopiperazin und DDLL-Methionindiketopiperazin entsteht mit einem Anteil von bis zu 60 Mol% DLLD-Methionindiketopiperazin am Gesamtgehalt der DLLD- und DDLL-Methionylmethioninäquivalente im Reaktionsgemisch und
j. diese dann in Schritt b. zurückführt.

Dabei werden in Schritt d. vorzugsweise 0,9 bis 1,5 Moleq, besonders bevorzugt 1,0 bis 1,2 Moleq Base bezogen auf den Gesamtgehalt an DLLD- und DDLL-Methionyl-methioninäquivalenten im Reaktionsgemisch eingesetzt. Diese Verfahrensweise bewirkt eine praktisch quantitative Hydrolyse der DKP-Vorstufe zum Dipeptid.

Als Base in Schritt d. werden vorzugsweise Alkalimetallhydroxide verwendet, besonders bevorzugt NaOH oder KOH, weil diese eine ausreichend hohe Alkalität und Löslichkeit aufweisen, es sich dabei um relativ preiswerte aber gleichzeitig in hoher Qualität verfügbare Industriechemikalien handelt, die darüber hinaus im Hinblick auf eventuell im Endprodukt verbleibende Spuren völlig unproblematisch sind,

Die in Schritt e. verwendete Kohlensäure bzw. das verwendete CO₂/H₂O-Gemisch bildet bei der Neutralisation mit NaOH oder KOH die entsprechenden Natrium- bzw. Kaliumhdrogencarbonate und -carbonate, welche gut bis sehr gut in Wasser löslich sind und sich in der Abtrennung des nur wenig wasserlöslichen Produkts DLLD-Methionylmethionin als besonders hilfreich erwiesen haben, da sie bei der Trennung des Met-Met-Niederschlags von der Mutterlauge in dieser verbleiben und somit praktisch vollständig abgetrennt werden können (Aussalzeffekt). Bei der Wahl der Lauge ist die Löslichkeit der Alkalibicarbonate und -carbonate zu berücksichtigen. So ist die Löslichkeit der Kaliumsalze um ungefähr den Faktor drei höher als die der Natriumsalze. Es muss also sichergestellt sein, dass es zu keinem Copräzipitat der anorganischen Salze mit Methionyl-methionin kommt. Dementgegen ist der Einsatz von NaOH aufgrund der Rohstoffpreise wirtschaftlicher.

In Schritt f. wird vorzugsweise bis zu einer Temperatur technisch und wirtschaftlich realisierbaren Temperatur von 30 °C abgekühlt, bevorzugt auch zu tieferen Temperaturen von 10 bis 20°C. Das hat den Effekt, dass höchstens kleine Anteile an noch vorhandenem DDLL- Met-Met mit ausfallen, so dass im Produkt nur noch ein Anteil von deutlich weniger als 5 % des unerwünschten Diastereomeren DDLL- Met-Met verbleibt.

Der Prozess wird vorzugsweise so gefahren, dass die in Schritt f. erzeugte Suspension mindestens 1 Gew%, jedoch nicht mehr als 15 Gew.% Gesamtgehalt an DDLL- und DLLD -Methioninylmethionin enthält, Auf diese Weise wird erreicht, dass relativ gut filtrierbare Kristalle, hauptsächlich bestehend aus DLLD-Met-Met erzeugt werden können. Das DDLL-Met-Met verbleibt mit über 95% in Lösung. Eine solche Trennung ist mit dem Ausgangsprodukt, dem cyclischen Dipeptid DKP, das ein Verhältnis von DLLD-DKP / DDLL-DKP von ca. 55 / 45 aufweist, nicht möglich wie bereits ausgeführt.

Der in Schritt g. abgetrennten Feststoffanteil wird weiter gereinigt und/oder getrocknet, wodurch ein pulverförmiges, weitgehend diastereomerenreines DLLD-Methionylmethionin erhalten wird, welches direkt geeignet ist zur Verwendung als Futtermittelzusatzstoff für Aquakulturen.

Ein weiterer Gegenstand der Erfindung ist somit ein

Pulverförmiges, weitgehend diastereomerenreines DLLD-Methionylmethionin, erhältlich nach einem der oben beschriebenen erfindungsgemäßen Verfahren, welches geeignet ist zur Verwendung als Futtermittelzusatzstoff für Aquakulturen.

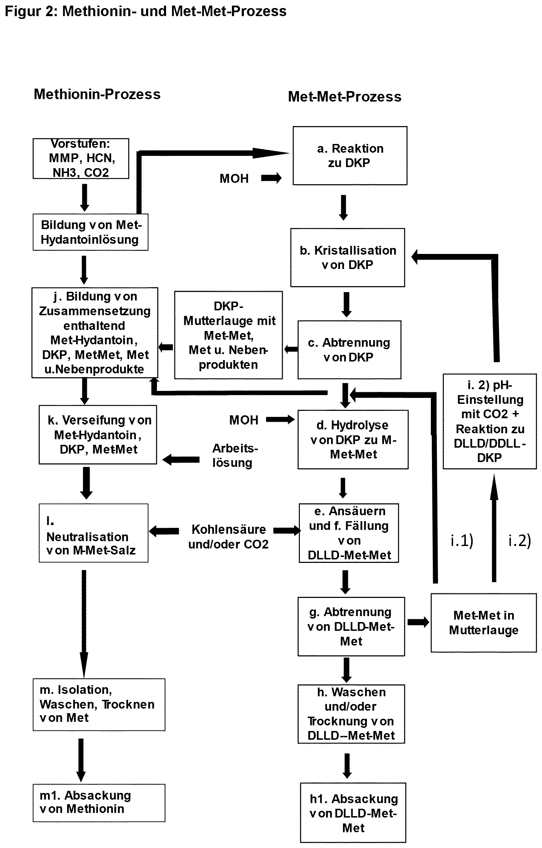

Ein weiterer Gegenstand der vorliegenden Erfindung und mit ihr in direktem Zusammenhang stehend ist schließlich das folgende Gesamtverfahren (vgl. Figur 2) zur (gleichzeitigen) Herstellung von diastereomerenreinem DLLD-Methionylmethionin und DL-Methionin dadurch gekennzeichnet, dass man (vgl. Schrittfolge des Ablaufschemas in Figur 2)
a. eine Methioninhydantoin enthaltende wässrige Lösung, zusammen mit Alkalibase umsetzt zu Methionindiketopiperazin (DKP, I) ,
b. das DKP aus a. durch Einengen oder Abkühlen der Reaktionslösung aus a. zur Kristallisation bringt,
c. das auskristallisierte DKP aus b. von der DKP-Mutterlauge abtrennt,
d. das abgetrennte DKP aus c. in Wasser einmischt und das Gemisch durch Zugabe entsprechender Mengen Alkali (M) auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension Methionyl-methionin-Alkalisalz (M-Met-Met) hydrolysiert,
e. den pH-Wert der Lösung oder Suspension aus d., gemessen mit einer pH-Elektrode bei 20°C, durch Zufuhr entsprechender Mengen von Kohlensäure und/ oder CO2 auf auf pH 6 bis 9, vorzugsweise 6,4 bis 8,9 besonders bevorzugt 7,0 bis 8,5, ganz besonders bevorzugt 7,6 bis 8,5, insbesondere von 7,6 bis 8,2 gemäß absenkt,
f. durch Verdampfen von Wasser und/ oder Abkühlen eine Suspension erzeugt, die aus einem vorwiegend Methionylmethionin enthaltenden Feststoffanteil und einer Restmengen an Methionylmethionin enthaltenden Mutterlauge, besteht, man
g. den Feststoffanteil aus f. von der Mutterlauge abtrennt, und man
h. diesen wäscht und/oder trocknet, und dabei (hochreines) Methionylmethionin erhält und man
i. die Methionylmethionin enthaltende Mutterlauge aus g. der Zusammensetzung in Schritt j. zuführt (Weg1)) oder die Methionylmethionin enthaltende Mutterlauge aus g. (Weg 1)) oder dass man die Methionylmethionin enthaltende Mutterlauge aus g. zunächst durch Zufuhr von Kohlensäure und/oder CO2auf einen pH- Wert von 6,4 -7,5 bringt und dann bei einer Temperatur von 150 bis 220 °C mittels Cyclisierung umsetzt und in Schritt b (Weg 2)) zurückführt und man
j. die DKP-Mutterlauge aus c. enthaltend Methionindiketopiperazin,, Met-Met, Met und Nebenprodukte mit einer Methionin-Hydantoinlösung zusammenführt und
k. die in j. gebildete Zusammensetzung einer alkalischen Verseifung zum Alkalimethioninat unterzieht, vorzugsweise im entsprechenden Verseifungsteil einer Methioninproduktionsanlage, man
l. das Alkalimethioninat (M-Met) aus k. mit Säure neutralisiert, so dass eine Methionin-Suspension entsteht,
m. das Methionin aus der Suspension in k. isoliert, wäscht und trocknet.

Die in j. entstandene Zusammensetzung wird vorzugsweise gebildet durch Zusammenführung der DKP-Mutterlauge aus c. und der Methionin-Hydantoinlösung aus dem Methioninprozess im Gewichtsverhältnis von 1 :1 bis 1 :1000, vorzugsweise von 1 : 3 bis 1 : 100, besonders bevorzugt von 1:5 bis 1 : 20 gefahren. Hierbei wurde auch im Dauerbetrieb eine stabile kontinuierliche Fahrbarkeit der erfindungsgemäßen Kombination beider Teilverfahren zum Gesamtverfahren und damit dessen hohe Robustheit gezeigt und dabei eine sehr gute Produktqualität der beiden Endprodukte DL-Methionin und DLLD-Met-Met erhalten, was ebenfalls ein großer Vorteil ist.

Darüber hinaus bedeutet die breite Variationsmöglichkeit bei den Gewichtsverhältnissen der zusammenzuführenden Prozesslösungen DKP-Mutterlauge aus c. und der Methionin-Hydantoinlösung aus dem Methioninprozess auch eine relativ breite Variationsmöglichkeit bei den gleichzeitig produzierbaren Mengen an DL-Methionin und DLLD- Met-Met. Die überraschend hohe Flexibilität des erfindungsgemäßen Gesamtverfahrens ermöglicht es somit je nach Marktbedarf mehr oder weniger DL-Methionin bzw. DLLD- Met-Met zu produzieren, was von hohem wirtschaftlichen Wert ist.

Die Herstellung der unter a. genannten Methioninhydantoin enthaltenden wässrigen Lösung kann im Wesentlichen erfolgen nach den in EP 780370 A2 in Figur 1 bezüglich der Herstellung einer Methionin-Hydantoinlösung gemachten Angaben.

Die Schritte k. bis m. korrespondieren zu dem in EP 780370 A2 in Figur 2 + 3 jedoch allein bezogen auf die Methionin-Hydantoinlösung dargestellten Verfahrensabschnitt der Hydantoinverseifung, und die dort angegebenen Verseifungsbedingungen können im hier dargestellten Gesamtverfahren analog angewendet werden.

### Beschreibung der Figuren:

**Figur 1** **zeigt das Schema zur Herstellung DLLD-Methionyl-methionins**
   Das Schema umfasst die folgenden Schritte:
   a. Reaktion von Methioninhydantoin mit Alkalibase zu DLLD/DDLL-Met-DKP
   b. Kristallisation von DLLD/DDLL-Met-DKP
   c. Abtrennung von DLLD/DDLL-Met-DKP und Ausschleusung der DKP-Mutterlauge zur Methioninanlage
   d. alkalische Hydrolyse von DLLD/DDLL-Met-DKP zu DLLD/DDLL-Met-Met
   e. Ansäuern und
   f. Fällung von DLLD-Met-Met
   g. Abtrennung des Feststoffs DLLD -Met-Met
   h. Wäsche und/oder Trocknung von DLLD-Met-Met
      h1. Absackung von DLLD-Met-Met
   i. pH-Einstellung mit Kohlensäure und/oder CO2 auf 6,4- 7,5 und Reaktion und Epimerisierung von DDLL -Met in DDLL -Met-haltiger Mutterlauge zu DLLD/DDLL-Met-DKP
   j. Rückführung von DLLD/DDLL-Met-DKP in Schritt b.
**Figur 2** **zeigt ein Schema zum DL-Methionin-/ DLLD-Methionylmethionin-Prozess.**

Das Schema umfasst die folgenden Schritte (Bezugszeichenliste):

### Bildung einer wässrigen Lösung enthaltend Methioninhydantoin

- a.: Reaktion zu DKP ausgehend von vorstehender Methionin-Hydantoinlösung mit Hilfe von Alkalihydroxid (MOH)
- b.: Kristallisation von DKP aus a.
- c.: Abtrennung des auskristallisierten DKP aus b. von der DKP-Mutterlauge,
- d.: Hydrolyse des abgetrennten DKP aus c. mit Hilfe von Alkalihydroxid (MOH) zum Alkalimethioninat (M-Met-Met),
- e.: Ansäuern des Hydrolysats aus d. durch Säurezugabe unter Freisetzung von Methionylmethionin und
- f.: Fällung von Methionylmethionin
- g.: Abtrennung des ausgefällten Met-Met aus f. von der Met-Met-Mutterlauge
- h.: Waschen und/oder Trocknung von Methionylmethionin aus g.
- h1.: Absackung von Met-Met
- i.: 1) direkte Rückführung in j. oder 2) pH-Einstellung mit Kohlensäure und/oder CO2 auf 6,4-7,5 und Reaktion des Methionylmethionin in der Met-Met- Mutterlauge aus g. zu DKP und Rückführung in Schritt c.,
- j.: Zusammenführung der DKP-Mutterlauge aus c. enthaltend Methionindiketopiperazin (I), Met-Met, Met und Nebenprodukte mit einer Methionin-Hydantoinlösung zu einer Zusammensetzung
- k.: alkalische Verseifung der Zusammensetzung aus j. enthaltend Methioninhydantoin, DKP Methionylmethionin, Met und Nebenprodukte zu Alkalimethioninat im Verseifungsteil einer Methioninproduktionsanlage,
- I.: Neutralisation des Alkalimethioninat aus k. mit CO2/H2O, so dass eine Methionin-Suspension entsteht,
- m.: Isolation, Waschen und Trocknen von Methionin aus I.
- m1.: Absackung von Methionin

### Beispiele

Die Beispiele wurden, wenn nicht anders angegeben, wie in der allgemeinen Verfahrensbeschreibung und gemäß der jeweils zugehörigen Angaben in Tabelle 1 durchgeführt.

### A) Analytische Methoden

### 1. High Performance Liquid Chromatography (HPLC)

Die **Quantitative Bestimmung der Konzentrationen** von Met, Met-Met, Methionyl-DKP usw. erfolgte mit Standard-HPLC Methoden gegenüber einem externen Standard.

Die Untersuchungen wurden mit HPLC der Firma JASCO an einer geeigneten RP-Säule mit anschließender UV-Detektion bei 210 nm durchgeführt. Als Laufmittel diente ein phosphorsaures Acetonitril-Wasser-Gemisch. Bei einem Fluss von 1 mL/min wurden 10 µL der jeweiligen Probelösung injiziert. Die Kalibrierung erfolgte im Vorfeld durch die Injektion geeigneter Kalibrierlösungen entsprechender Referenzverbindungen aus dem Methionin- bzw. Met-Met-Verfahren, die Auswertung erfolgte durch Peakflächenvergleich mittels der externen Standardmethode. Die Durchführung der Standardmethode ist dem Fachmann bekannt.

### 2. Probengewinnung zur Bestimmung von Met-Met im Niederschlag:

Ein Raumteil des abfiltrierten Niederschlags aus der Carbonisierung wurde auf einer Laborfilternutsche gesammelt und mit zwei Volumenequivalenten Aceton übergossen und mit Hilfe eines reduzierten Drucks im Wasserstrahlvakuum abgesaugt. Dabei wurde der noch vorhandene und in Aceton etwas lösliche Anteil der am Niederschlag anhaftenden Mutterlauge weitgehend entfernt. Es wurde noch für 5 min weitergesaugt und dann in einem Trockenschrank bis zur Gewichtskonstanz getrocknet, so dass das restliche Aceton entfernt war. Anschließend wurde ein geeigneter Anteil des so vorbehandelten Niederschlags eingewogen, im HPLC-Laufmittel aufgenommen und mit weiterem HPLC-Laufmittel auf eine geeignete Konzentration herunterverdünnt und die so erhaltene Probenlösung wie oben erläutert in die HPLC injiziert.

### 3.Bestimmung des pH-Wertes

Der pH-Wert wurde mit Hilfe einer pH-Elektrode (=Glaselektrode) in wässriger Lösung und bei einer Temperatur von 20°C gemessen sofern nicht anders angegeben.

### B) Allgemeine Verfahrensbeschreibung der DKP-Synthese

Eine wässrige Lösung des Dipeptides Met-Met wurde nach Anpassung des pH-Wertes auf 4,5 oder 5 mit Hilfe von entweder Schwefelsäure oder Ätznatron für eine Zeitdauer von 30 - 120 min auf 150 - 180°C aufgeheizt. Nach Abkühlung auf 70°C durch Druckabsenkung wurde eine wässrige Suspension erhalten. Nach Abfiltrieren und Waschen des Filterkuchens mit Wasser wurde ein Feststoff erhalten, der hauptsächlich DKP enthielt, das aus einer Mischung von DLLD-DKP und DDLL-DKP in einem Verhältnis von im günstigen Fall 50/50 oder größer bestand unabhängig von der stereochemischen Konfiguration des Ausgangsmaterials Met-Met.

### C) Gewinnung der Alkali-DLLD/DDLL-Met-Met-Lösung durch alkalische Hydrolyse von DKP

Zu 7,70 kg Wasser wurden 1,54 kg 50%ige Natronlauge (19 mol, 1,3 eq.) zugegeben und auf 95°C erhitzt. Temperaturkontrolliert wurden 4,68 kg DKP (83%, 14,8 mol) dosiert, so dass die Temperatur nicht unter 90°C sank. Nach vier Stunden war die gesamte DKP-Menge zugegeben. Während der Zugabe verändert sich die Farbe des Reaktionsgemischs über gelb nach braun. Abschließend wurde noch eine Stunde auf 95°C nachtemperiert; dabei sank der DKP-Gehalt unter 0,2% (HPLC-Kontrolle). 13,9 kg NaMetMet-Lösung (13,5 mol, 91% Ausbeute) wurden in einen Vorlagebehälter für die Kristallisation transferiert.

Das analoge Verseifungsprodukt KMetMet-Lösung wurde analog der Gewinnung von NaMetMet-Lösung durch entsprechende Verwendung von Kalilauge zur Hydrolyse erzeugt.

### D) Ansäuern/Neutralisieren der Alkali-DLLD/DDLL-Met-Met-Lösung und Fällung von Met-Met Vergleichsbeispiel, Ansäuern mit Mineralsäure H2SO4:

Zur Kristallisation des Dipeptids durch Mineralsäure (H2SO4) wurde die Na- DLLD/DDLL-MetMet-Lösung mit H2SO4 auf pH 5,2 gestellt. Dazu wurden 11,6 kg einer im oben beschriebenen Ansatz isolierten Mutterlauge (0,14 mol NaMetMet) im Reaktor vorgelegt. Dann wurden 13,9 kg NaMetMet-Lösung (13,5 mol; Fluss von 6 kg/h) pH-gesteuert mit 0,51 kg einer konz. Schwefelsäure (97%; 0,50 mol) in einem Reaktor bei konstantem pH-Wert (Soll pH 5,2) zusammengefahren. Die Temperatur während der Neutralisation betrug max. 40°C. Der pH-Wert wurde während der Neutralisation im Bereich zwischen 5,0 und 5,5 gehalten. Nach zwölf Stunden Rühren bei 20°C wurde die entstandene weiße Suspension abfiltriert und mit 2 L Wasser gewaschen. Auf diese Weise wurden 4,74 kg weißes MetMet mit einem Wassergehalt von 25% abgetrennt. Nach Trocknung über Nacht im Stickstoffstrom bei 80°C wurden 3,56 kg (12,7 mol) weißes Met-Met erhalten mit einem Verhältnis von 65/35 DLLD-Met-Met / DDLL-Met-Met, was einer Ausbeute von 94% bezogen auf das eingesetzte NaMetMet entsprach.

### Beispiele 1 bis 2, erfindungsgemäße Neutralisation mittels Carbonisierung (siehe Tabelle 1)

Die gemäß A) erzeugten Gemische von DLLD/DDLL-Met-Met-Kalisalzen wurde außerdem gemäß der Angaben in Tabelle 1 mit CO2 bis auf pH Werte im Bereich 6 bis 9 DLLD-Met-Met neutralisiert, so dass ein überwiegend DLLD-Met-Met enthaltender Feststoff als Niederschlag ausfiel. Dieser wurde abfiltriert, gewaschen und getrocknet. Die molaren Anteile von DLLD-Met-Met im Niederschlag von 96,4 bis 97,2 mol % - wie in Tab. 1 angegeben.

### Kommentar zu Tabelle 1

In Beispiel 1 wurde nach Ansäuern (Carbonisieren) der Alkali-Met-Met enthaltenden DKP-Hydrolysats ein molares Met-Met-End-verhältnis im Niederschlag nach Carbonisierung von **97,2 / 2,6** DLLD / DDLL-Met-Met erhalten, was eine sehr gute Selektivität und einen entsprechend hohen Reinheitsgrad widerspiegelt, so dass die oben gestellte Aufgabe in einfacher, sehr guter und überraschender Weise gelöst werden konnte.

Im Vergleich dazu wurde nach Ansäuern des Alkali-Met-Met enthaltenden DKP-Hydrolysats auf pH 5,2 mit der starken Mineralsäure H2SO4 ein molares Met-Met-End-verhältnis im Niederschlag von nur **65,0 /35,0** DLLD / DDLL-Met-Met erhalten.

Die überraschend große Selektivität der Fällung des gewünschten Diastereomeren DLLD-Met-Met scheint dabei auf ein Zusammenwirken des desspeziellen pH-Bereichs von 6 bis 9 und der speziellen Matrixzusammensetzung der carbonisierten DKP-Hydrolysats zurückzuführen sein.

**Tabelle1: Überblick über die Beispiele**

| **Beispiel** | **Ausgangsmaterial/ Art der Umsetzung** | **Ansäuren bzw. Carbonisierung Zeit [min]** | **Temp. [°C] beim Ansäuern bzw. in Carbonisierung** | **pH bei 20°C nach Ansäuern /Carbonisierung** | **Gesamt Met-Met -Konz. [Gew%] in Mutterlauge** | **Met-Met-Startverhältnis DLLD / DDLL in Mutterlaug e nach Verseifung [Mol/Mol]** | **Met-Met-End-verhältnis im Niederschlag nach Carbonisierung DLLD / DDLL [Mol/Mol]** | **Met-Met-End-verhältnis in Mutterlauge nach Carbonisierung DLLD / DDLL [Mol/Mol]** |
|---|---|---|---|---|---|---|---|---|
| **Vergleic h** | **Ansäuern von DKP-Hydrolysat (= Met-Met-Alkalisalzlösung /-Suspension) mit H2SO4** | **90** | **Max.40** | **5,2** | **17,2** | **60,0/40,0** | **65,0 /35,0** | **16,0/84,0** |
| **1** | **Ansäuern von DKP-Hydrolysat (= Met-Met-Alkalisalzlösung oder - Suspension) mit Kohlensäure und/oder CO2** | | **20** | **8,0** | **17,2** | **65,0 / 35,0** | **97,4 /2,6** | **28,1/70,9** |
| **2** | **⁻"** | **40** | **20** | **7,6** | **18,0** | **54,9/44,7** | **96,9/3,1** | |

## Patentansprüche

1. Verfahren zur Gewinnung von diastereomerenreinem DLLD-Methionylmethionin, **dadurch gekennzeichnet, dass** man eine das Natrium- und/oder Kaliumsalz von DLLD-Methionyl-methionin und das Natrium- und/oder Kaliumsalz von DDLL-Methionyl-methionin enthaltende wässrige Lösung oder Suspension mit entsprechenden Mengen Kohlensäure und/oder CO₂ versetzt (carbonisiert), so dass ein pH-Wert von 6 bis 9, gemessen mit einer Glaselektrode bei der sich einstellenden Temperatur erreicht wird, wobei ein überwiegend DLLD-Methionylmethionin enthaltender Niederschlag mit einem DLLD-Methionylmethionin-Anteil von mindestens 70 mol % gemessen am Gesamtgehalt von DLLD-Methionylmethionin und DDLL-Methionylmethionin ausfällt, der von der Mutterlauge, die DDLL-Methionylmethionin in angereicherter Form enthält, abgetrennt wird.

2. Verfahren gemäß Anspruch 1, **dadurch gekennzeichnet, dass** man bis zum Erreichen eines pH-Wertes von 6,4 bis 8,9 vorzugsweise 7,0 bis 8,5, besonders bevorzugt 7,6 bis 8,5, ganz besonders bevorzugt von 7,6 bis 8,2 carbonisiert.

3. Verfahren gemäß einem der Ansprüche 1 oder 2, **dadurch gekennzeichnet, dass** man so lange carbonisiert, bis ein DLLD-Methionylmethionin-Anteil von mindestens 80 mol %, vorzugsweise 90 % im Niederschlag erhalten wird.

4. Verfahren gemäß einem der Ansprüche 1 bis 3, **dadurch gekennzeichnet, dass** man die Carbonisierung bei einer Temperatur von 20 bis 50 °C, vorzugsweise bei 25 bis 40°C durchführt.

5. Verfahren zur Herstellung von diastereomerenreinem DLLD-Methionylmethionin, **dadurch gekennzeichnet, dass** man
a. Methionin-Hydantoin zusammen mit Alkalibase umsetzt zu einem Diastereomerengemisch aus DLLD-Methionindiketopiperazin und DDLL-Methionindiketopiperazin (DLLD-DKP und DDLL-DKP)
b. das DLLD-DKP und DDLL-DKP aus a. durch Einengen oder Abkühlen der Reaktionslösung aus a. zur Kristallisation bringt
c. das auskristallisierte DLLD-DKP und DDLL-DKP abtrennt von der DKP-Mutterlauge
d. das abgetrennte DLLD-DKP und DDLL-DKP in Wasser einmischt und das Gemisch durch Zugabe entsprechender Mengen Alkali auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DLLD-Methionylmethionin- und DDLL-Methionylmethionin-Alkalisalz hydrolysiert,
e. den pH-Wert der Lösung oder Suspension aus d., gemessen mit einer Glaselektrode bei der sich einstellenden Temperatur, durch Zugabe entsprechender Mengen Kohlensäure und/oder CO₂ auf pH 6 bis 9, vorzugsweise 6,4 bis 8,9 besonders bevorzugt 7,0 bis 8,5, ganz besonders bevorzugt 7,6 bis 8,5, insbesondere von 7,6 bis 8,2 gemäß Anspruch 1 bis 4 absenkt,
f. durch Verdampfen von Wasser und/ oder Abkühlen eine Suspension erzeugt, die aus einem Feststoffanteil, der vorwiegend DLLD-Methionylmethionin enthält, und Mutterlauge, die vorwiegend DDLL-Methionylmethionin enthält, besteht, man
g. den Feststoffanteil aus f. von der Mutterlauge abtrennt, und man
h. ihn wahlweise wäscht und/oder trocknet, und dabei diastereomerenreines DLLD-Methionylmethionin erhält und man
i. die verbleibende DDLL-Methionylmethionin enthaltende Mutterlauge aus g. durch Zufuhr entsprechender Mengen Kohlensäure und /oder CO2 auf einen pH-Wert von 6,4 bis 7,5 solange bei einer Temperatur von 150 bis 220°C, vorzugsweise bei 170 bis 210 °C umsetzt, bis eine wässrige Lösung oder Suspension enthaltend mindestens einen Anteil von 50 Mol %, insbesondere 60 Mol% DLLD-Methionindiketopiperazin bezogen auf den Gesamtgehalt an DLLD- und DDLL-Methionindiketopiperazin im Reaktionsgemisch entstanden ist, und man
j. diese dann in Schritt b. zurückführt

6. Verfahren gemäß Anspruch 5, **dadurch gekennzeichnet, dass** man in Schritt d. 0,9 bis 1,5 Moleq, vorzugsweise 1,0 bis 1,2 Moleq Base bezogen auf den Gesamtgehalt an DLLD- und DDLL-Methionyl-methioninäquivalenten im Reaktionsgemisch einsetzt.

7. Verfahren gemäß Anspruch 5 oder 6, **dadurch gekennzeichnet, dass** man in Schritt d. Alkalimetallhydroxide, vorzugsweise NaOH oder KOH als Base verwendet.

8. Verfahren gemäß einem der Ansprüche 1 bis 4, oder 5e. bis 7, **dadurch gekennzeichnet, dass** man in Schritt e. CO2 bei einem Druck von 1 bis 7 bara bar verwendet.

9. Verfahren gemäß einem der Ansprüche 5 bis 8, **dadurch gekennzeichnet, dass** man in Schritt f. bis zu einer Temperatur von > 50 °C abkühlt.

10. Verfahren gemäß einem der Ansprüche 5 bis 9, **dadurch gekennzeichnet, dass** die in Schritt f. erzeugte Suspension mindestens 1 Gew%, jedoch nicht mehr als 15 Gew.% Gesamtgehalt an DDLL- und DLLD -Methionyl-methionin enthält.

11. Pulverförmiges diastereomerenreines DLLD-Methionylmethionin, erhältlich nach einem Verfahren gemäß einem der Ansprüche 5 bis 11 zur Verwendung als Futtermittelzusatzstoff für Aquakulturen.

12. Verwendung von diastereomerenreinem DLLD-Methionyl-methionin gemäß Anspruch 11 als Futtermittelzusatzstoff für Aquakulturen.

13. Verfahren zur gleichzeitigen Herstellung von diastereomerenreinem DLLD-Methionylmethionin und DL-Methionin **dadurch gekennzeichnet, dass** man
a. eine wässrige Lösung enthaltend Methioninhydantoin, zusammen mit Alkalibase umsetzt zu Methionindiketopiperazin (DKP, I),
b. das DKP aus a. durch Einengen oder Abkühlen der Reaktionslösung aus a. zur Kristallisation bringt,
c. das auskristallisierte DKP aus b. von der DKP-Mutterlauge abtrennt,
d. das abgetrennte DKP aus c. in Wasser einmischt und das Gemisch durch Zugabe entsprechender Mengen Alkali auf einen pH-Wert von 10 bis 14, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension Methionyl-methionin-Alkalisalz hydrolysiert,
e. den pH-Wert der Lösung oder Suspension aus d., gemessen mit einer pH-Elektrode bei 20°C, durch Zufuhr entsprechender Mengen von Kohlensäure und/ oder CO2 auf pH 6 bis 9, vorzugsweise 6,4 bis 8,9 besonders bevorzugt 7,0 bis 8,5, ganz besonders bevorzugt 7,6 bis 8,5, insbesondere von 7,6 bis 8,2 absenkt gemäß einem der Ansprüche 1 bis 4,
f. durch Verdampfen von Wasser und/ oder Abkühlen eine Suspension erzeugt, die aus einem vorwiegend DLLD-Methionylmethionin enthaltenden Feststoffanteil und einer Restmengen an DDLL-Methionylmethionin enthaltenden Mutterlauge, besteht, man
g. den Feststoffanteil aus f. von der Mutterlauge abtrennt, und man
h. diesen wäscht und/oder trocknet, und dabei diastereomerenreines DLLD-Methionylmethionin erhält und man
i. die Methionylmethionin enthaltende Mutterlauge aus g. der Zusammensetzung in Schritt j. zuführt (Weg 1)) oder dass man die Methionylmethionin enthaltende Mutterlauge aus g. zunächst durch Zufuhr von Kohlensäure und/oder CO2 den pH-Wert auf 6,4 -7,5 bringt und dann bei einer Temperatur von 150 bis 220 °C mittels Cyclisierung umsetzt, so dass eine wässrige Lösung oder Suspension enthaltend Methionindiketopiperazin entsteht und man diese dann in Schritt b. zurückführt (Weg 2)), und man
j. die DKP-Mutterlauge, enthaltend Methionindiketopiperazin, Nebenprodukte, gegebenenfalls Met-Met und Met aus c. mit einer Methionin-Hydantoinlösung zusammenführt und
k. die in j. gebildete Zusammensetzung einer alkalischen Verseifung zum Alkalimethioninat unterzieht im entsprechenden Verseifungsteil einer Anlage zur Herstellung von Methionin, man
l. das Alkalimethioninat aus k. mit CO₂ neutralisiert, so dass eine Methionin-Suspension entsteht,
m. das Methionin aus der Suspension in I. isoliert, wäscht und trocknet.

14. Verfahren nach Anspruch 12, **dadurch gekennzeichnet, dass** man den pH -Wert in Schritt d. auf 13 bringt.

15. Verfahren nach einem der Ansprüche 13 bis 14, **dadurch gekennzeichnet, dass** die in j. entstandene Zusammensetzung gebildet wird durch Zusammenführung der DKP-Mutterlauge aus c. und der Methionin-Hydantoinlösung aus dem Methioninprozess im Gewichtsverhältnis von 1 :1 bis 1 :1000, vorzugsweise von 1 : 3 bis 1 : 100, besonders bevorzugt von 1:5 bis 1 : 20.
